(19) Europäisches Patentamt
European Patent Office
Office européen des brevets

(11) Veröffentlichungsnummer: **0 222 694**
**A2**

# (12) EUROPÄISCHE PATENTANMELDUNG

(21) Anmeldenummer: 86810407.6

(22) Anmeldetag: 08.09.86

(51) Int.Cl.[4]: **C 07 D 295/08**
C 07 D 211/14, C 07 D 265/30
C 07 D 303/04, C 07 D 303/08
C 07 D 303/22, A 01 N 33/08

(30) Priorität: 13.09.85 CH 3989/85

(43) Veröffentlichungstag der Anmeldung:
20.05.87 Patentblatt 87/21

(84) Benannte Vertragsstaaten:
AT BE CH DE FR GB IT LI LU NL SE

(71) Anmelder: CIBA-GEIGY AG
Klybeckstrasse 141
CH-4002 Basel(CH)

(72) Erfinder: Riebli, Peter
Bünten 17
D-4446 Buckten(CH)

(54) **Mikrobizide.**

(57) Propan-2-ol-Derivate der Formel I

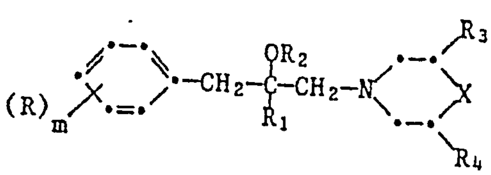

(I),

worin X Sauerstoff oder $CH_2$ bedeutet und die Substituenten R, $R_1$ und $R_2$ die hierin angegebene Bedeutung haben, während $R_3$ und $R_4$ unabhängig Wasserstoff oder $C_1$-$C_4$-Alkyl darstellen und m einen Index 0 bis 3 bedeutet, besitzen samt ihren Säureadditionssalzen mikrobizide Wirkung. Sie wirken besonders gegen pflanzenpathogene Erreger. In Form üblicher Mittel lassen sie sich zum Schutze von Kulturpflanzen gegen Krankheitsbefall einsetzen.

EP 0 222 694 A2

0222694

CIBA-GEIGY AG
Basel (Schweiz)

5-15506/+

Mikrobizide

Die vorliegende Anmeldung betrifft 1-Amino-3-phenyl-propan-2-ol-Derivate der nachstehenden Formel I sowie deren Säureadditionssalze. Die Erfindung betrifft ferner die Herstellung dieser Substanzen sowie mikrobizide Mittel, die als Wirkstoff mindestens eine dieser Verbindungen enthalten. Die Erfindung betrifft auch die Verwendung der Wirkstoffe zur Bekämpfung schädlicher Mikroorganismen, insbesondere pflanzenpathogener Fungi.

Die erfindungsgemässen Verbindungen haben die Formel I

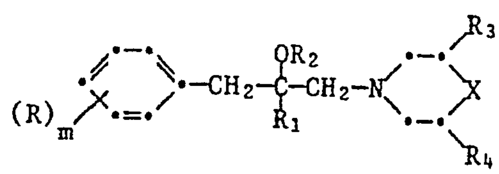

(I),

worin

m = 0 bis 3,

R = Halogen; $C_1$-$C_6$-Alkyl; ein- oder mehrfach durch Halogen und/oder $C_1$-$C_3$-Alkoxy substituiertes $C_1$-$C_6$-Alkyl; $C_1$-$C_4$-Alkoxy; ein- oder mehrfach durch Halogen substituiertes $C_1$-$C_4$-Alkoxy; $C_3$-$C_6$-Cycloalkyl;

$R_1$ = $C_1$-$C_4$-Alkyl; ein- oder mehrfach durch Halogen substituiertes $C_1$-$C_4$-Alkyl,

$R_2$ = $C_1$-$C_6$-Alkyl; ein- oder mehrfach durch Halogen und/oder $C_1$-$C_3$-Alkoxy substituiertes $C_1$-$C_6$-Alkyl; $C_2$-$C_4$-Alkenyl; 2-Propinyl; 3-Halogen-2-propinyl; Benzyl; ein- oder mehrfach durch Halogen, $C_1$-$C_4$-Alkoxy, $C_1$-$C_4$-Alkyl, $C_1$-$C_4$-Haloalkyl oder $C_1$-$C_4$-Haloalkoxy substituiertes Benzyl,

oder $-\overset{O}{\overset{\|}{C}}-R_5$ oder $-\overset{O}{\overset{\|}{C}}NH-R_6$ bedeuten, wobei

$R_5$ = $C_1$-$C_6$-Alkyl; ein- oder mehrfach durch Halogen und/oder $C_1$-$C_3$-Alkoxy substituiertes $C_1$-$C_6$-Alkyl; $C_2$-$C_4$-Alkenyl; Phenyl; Benzyl; ein- oder mehrfach durch Halogen, $C_1$-$C_4$-Alkyl, $C_1$-$C_4$-Alkoxy, $C_1$-$C_4$-Haloalkyl, $C_1$-$C_4$-Haloalkoxy substituiertes Phenyl oder Benzyl und

$R_6$ = $C_1$-$C_6$-Alkyl; $C_2$-$C_4$-Alkenyl; Phenyl; ein- oder mehrfach durch Halogen, $C_1$-$C_4$-Alkyl, $C_1$-$C_4$-Alkoxy, $C_1$-$C_4$-Haloalkoxy, $C_1$-$C_4$-Haloalkyl substituiertes Phenyl sind, und

$R_3$ und $R_4$ unabhängig H oder $C_1$-$C_4$-Alkyl darstellen, während

X = Sauerstoff oder $CH_2$ bedeutet,

unter Einschluss ihrer Säureadditionssalze.

Unter dem Begriff Alkyl selbst oder als Bestandteil eines anderen Substituenten wie Alkoxy, Haloalkyl, Haloalkoxy etc., sind je nach Zahl der angegebenen Kohlenstoffatome beispielsweise die folgenden geradkettigen oder verzweigten Gruppen zu verstehen: Methyl, Ethyl, Propyl, Butyl, Pentyl, Hexyl sowie ihre Isomeren, wie z.B. Isopropyl, Isobutyl, tert.-Butyl, Isopentyl usw. Halogen und Halo stehen für Fluor, Chlor, Brom oder Jod. Haloalkyl steht somit für einen einfach bis perhalogenierten Alkylrest, wie z.B. $CHCl_2$, $CH_2F$, $CCl_3$, $CH_2Cl$, $CHF_2$, $CHFCH_3$, $CH_2CH_2Br$, $C_2Cl_5$, $CH_2Br$, CHBrCl usw., vorzugsweise für $CF_3$. Alkenyl steht z.B. für Propenyl-(1), Allyl, Butenyl-(1), Butenyl-(2) oder Butenyl-(3). $C_3$-$C_6$-Cycloalkyl steht wahlweise für Cyclopropyl, Methylcyclopropyl, Cyclobutyl, Cyclopentyl oder Cyclohexyl.

Beispiele salzbildender Säuren sind anorganische Säuren: Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, Phosphorige Säure, Salpetersäure und organische Säuren wie Essigsäure, Trifluoressigsäure, Trichloressigsäure, Propionsäure, Weinsäure, Glykolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure. Diese Säuren werden nach an sich bekannten Methoden an die freien Verbindungen der Formel I addiert.

Je nach Bedeutung des Substituenten X stellen die Verbindungen 1-Morpholino-3-phenyl-propan-2-ol-derivate (I-A) oder 1-Piperidino-3-phenyl-propan-2-ol-derivate (I-B) dar.

Die Verbindungen der Formel I sind bei Raumtemperatur stabil. Sie lassen sich auf dem Agrarsektor oder verwandten Gebieten präventiv und kurativ zur Bekämpfung von pflanzenschädigenden Mikroorganismen einsetzen. Die erfindungsgemässen Wirkstoffe der Formel I zeichnen sich in weiten Anwendungskonzentrationen durch eine sehr gute fungizide Wirkung und problemlose Anwendung aus.

Aufgrund ihrer ausgeprägten mikrobiziden Wirkung sind folgende Substanzgruppen unter Einschluss ihrer Säureadditionssalze bevorzugt.

I-A (Morpholin-Reihe):

Verbindungen der Formel I mit X = O,
worin m = 1 oder 2, und

R = Halogen, $C_1$-$C_4$-Alkyl, $C_1$-$C_2$-Alkoxy, ein- bis dreifach durch Halogen oder $C_1$-$C_3$-Alkoxy substituiertes Methyl; ein- bis dreifach durch Halogen substituiertes $C_1$-$C_2$-Alkoxy; $C_3$-$C_6$-Cycloalkyl;

$R_1$ = Methyl, Ethyl oder Isopropyl, die unsubstituiert oder ein- oder mehrfach durch Fluor oder Chlor substituiert sind; während

$R_2$ die für Formel I gegebene Bedeutung hat, und

$R_3$ und $R_4$ Wasserstoff, Methyl oder Ethyl darstellen (Gruppe I-A1).

Insbesondere sind jene Verbindungen aus der Gruppe I-A1 bevorzugt, worin

$R_2$ = $C_1$-$C_4$-Alkyl, ein- oder mehrfach durch Halogen substituiertes $C_1$-$C_4$-Alkyl, durch eine $C_1$-$C_3$-Alkoxy-Gruppe substituiertes $C_1$-$C_2$-Alkyl; $C_2$-$C_4$-Alkenyl; 2-Propinyl; Benzyl oder bis zu dreifach durch Chlor und/oder Methyl substituiertes Benzyl, oder -CO-$R_5$ bedeutet, wobei

$R_5$ = $C_1$-$C_4$-Alkyl, ein- oder mehrfach durch F, Cl oder Br substituiertes $C_1$-$C_3$-Alkyl, durch eine $C_1$-$C_3$-Alkoxy-Gruppe substituiertes $C_1$-$C_3$-Alkyl; Allyl oder Methylallyl; oder Phenyl darstellt, das bis zu dreifach durch Halogen, Methyl, Methoxy oder $CF_3$ substituiert sein kann. (Gruppe I-A2).

Eine der bevorzugten Untergruppen innerhalb der Gruppe I-A1 sind solche, worin m = 1 oder 2,

R = Fluor, Chlor oder Brom, Methyl, Ethyl, Isopropyl oder tert.-Butyl, $C_1$-$C_2$-Alkoxy, ein- bis dreifach durch Fluor und/oder Chlor substituiertes Methyl, durch $C_1$-$C_3$-Alkoxy substituiertes Methyl oder ein- bis dreifach durch Fluor oder Chlor substituiertes $C_1$-$C_2$-Alkoxy oder $C_3$-$C_6$ Cycloalkyl ist;

$R_1$ = Methyl, Ethyl oder $CF_3$,

$R_2$ = $C_1$-$C_4$-Alkyl, ein- bis dreifach durch Fluor oder Chlor substituiertes $C_1$-$C_4$-Alkyl, durch $C_1$-$C_3$-Alkoxy substituiertes $C_1$-$C_4$-Alkyl; $C_2$-$C_4$-Alkenyl; 2-Propinyl; Benzyl; ein- bis dreifach durch Chlor und/oder Methyl substituiertes Benzyl, oder -CO-$R_5$ oder -CONH$R_6$, wobei

$R_5$ = Methyl, Ethyl, Isopropyl, ein- bis dreifach durch F oder Cl substituiertes $C_1$-$C_3$-Alkyl, durch Methoxy, Ethoxy oder Isopropoxy substituiertes Methyl oder Ethyl; Allyl oder Methylallyl; oder Phenyl darstellt, das bis zu dreifach durch Halogen, Methyl, Methoxy oder $CF_3$ substituiert sein kann; und

$R_6$ = $C_1$-$C_6$-Alkyl oder unsubstituiertes oder bis zu dreifach durch Halogen, Methyl, Methoxy oder $CF_3$ substituiertes Phenyl bedeutet; und

$R_3$ und $R_4$ Wasserstoff oder Methyl darstellen. (Gruppe I-A3).

Wenn unter den Verbindungen der verschiedenen Gruppen Ia (X = Sauerstoff) $R_3$ und $R_4$ je $C_1$-$C_4$-Alkyl bedeuten, so sind als Wirkstoffe die cis-ständigen Isomeren bevorzugt.

I-B (Piperidin-Reihe):

Bevorzugt sind ferner
Verbindungen der Formel I mit X = $CH_2$,
worin m = 1 oder 2, und

R = Halogen, $C_1$-$C_4$-Alkyl, $C_1$-$C_2$-Alkoxy, ein- bis dreifach durch Halogen oder $C_1$-$C_3$-Alkoxy substituiertes Methyl; ein- bis dreifach durch Halogen substituiertes $C_1$-$C_2$-Alkoxy; oder $C_3$-$C_6$-Cycloalkyl bedeutet;

$R_1$ = Methyl, Ethyl oder Isopropyl, die unsubstituiert oder ein- oder mehrfach durch Fluor oder Chlor substituiert sind; während

$R_2$ die für Formel I gegebene Bedeutung hat, und

$R_3$ und $R_4$ Wasserstoff, Methyl oder Ethyl darstellen (Gruppe I-B1).

Insbesondere sind jene Verbindungen aus der Gruppe I-B1 bevorzugt, worin

$R_2$ = $C_1$-$C_4$-Alkyl, ein- oder mehrfach durch Halogen substituiertes $C_1$-$C_4$-Alkyl, durch eine $C_1$-$C_3$-Alkoxy-Gruppe substituiertes $C_1$-$C_2$-Alkyl; $C_2$-$C_4$-Alkenyl; 2-Propinyl; Benzyl oder bis zu dreifach durch Chlor und/oder Methyl substituiertes Benzyl, oder -CO-$R_5$ bedeutet, wobei

$R_5$ = $C_1$-$C_4$-Alkyl, ein- oder mehrfach durch F, Cl oder Br substituiertes $C_1$-$C_3$-Alkyl, durch eine $C_1$-$C_3$-Alkoxy-Gruppe substituiertes $C_1$-$C_3$-Alkyl; Allyl oder Methylallyl; oder Phenyl darstellt, das bis zu dreifach durch Halogen, Methyl, Methoxy oder $CF_3$ substituiert sein kann. (Gruppe I-B2).

Eine der bevorzugten Untergruppen innerhalb der Gruppe I-B1 sind solche, worin m = 1 oder 2,

R = Fluor, Chlor oder Brom, Methyl, Ethyl, Isopropyl oder tert.-Butyl, $C_1$-$C_2$-Alkoxy, ein- bis dreifach durch Fluor und/oder Chlor substituiertes Methyl, durch $C_1$-$C_3$-Alkoxy substituiertes Methyl oder ein- bis dreifach durch Fluor oder Chlor substituiertes $C_1$-$C_2$-Alkoxy; oder $C_3$-$C_6$-Cycloalkyl bedeutet;

$R_1$ = Methyl, Ethyl oder $CF_3$ ist;

$R_2$ = $C_1$-$C_4$-Alkyl, ein- bis dreifach durch Fluor oder Chlor substituiertes $C_1$-$C_4$-Alkyl, durch $C_1$-$C_3$-Alkoxy substituiertes $C_1$-$C_4$-Alkyl; $C_2$-$C_4$-Alkenyl; 2-Propinyl; Benzyl; ein- bis dreifach durch Chlor und/oder Methyl substituiertes Benzyl, oder -CO-$R_5$ oder -CONH$R_6$, wobei

$R_5$ = Methyl, Ethyl, Isopropyl, ein- bis dreifach durch F oder Cl substituiertes $C_1$-$C_3$-Alkyl, durch Methoxy, Ethoxy oder Isopropoxy substituiertes Methyl oder Ethyl; Allyl oder Methylallyl; oder Phenyl darstellt, das bis zu dreifach durch Halogen, Methyl, Methoxy oder $CF_3$ substituiert sein kann; und

$R_6$ = $C_1$-$C_6$-Alkyl oder unsubstituiertes oder bis zu dreifach durch Halogen, Methyl, Methoxy oder $CF_3$ substituiertes Phenyl bedeutet; und

$R_3$ und $R_4$ Wasserstoff oder Methyl darstellen. (Gruppe I-B3).

Bevorzugte Einzelverbindungen sind aus der Gruppe I-A:

1-(4H-Morpholino)-3-(4-tert.butylphenyl)-2-methyl-2-methoxypropan;

1-(4H-2,6-Dimethylmorpholino)-3-(4-tert.butylphenyl)-2-methyl-2-methoxypropan;

1-(4H-2,6-Dimethylmorpholino)-3-(4-tert.-butylphenyl)-2-methyl-2-ethoxypropan, und unter den beiden letztgenannten, insbesondere die <u>cis</u>-2,6-Dimethylmorpholino-Derivate;

1-(4H-2,6-Dimethylmorpholino)-3-(4-tert.-butylphenyl)-2-methyl-2-propargyloxypropan;

1-(4H-2,6-Dimethylmorpholino)-3-(4-tert.-butylphenyl)-2-methyl-2-allyloxypropan.

Bevorzugte Einzelverbindungen sind aus der Gruppe I-B:

1-(1H-Piperidino)-3-(4-tert.butylphenyl)-2-methyl-2-methoxypropan;

1-(1H-Piperidino)-3-(4-tert.butylphenyl)-2-methyl-2-ethoxypropan;

1-(1H-Piperidino)-3-(4-tert.butylphenyl)-2-ethyl-2-methoxypropan;

1-(1H-Piperidino)-3-(4-tert.butylphenyl)-2-methyl-2-allyloxypropan;

1-(1H-3,5-Dimethylpiperidino)-3-(4-tert.-butylphenyl)-2-methyl-2-methoxypropan;

1-(1H-3-Methylpiperidino)-3-(4-tert.butylphenyl)-2-methyl-2-methoxypropan.

Die Verbindungen der Formel I lassen sich

A) durch Reaktion einer Verbindung der Formel II

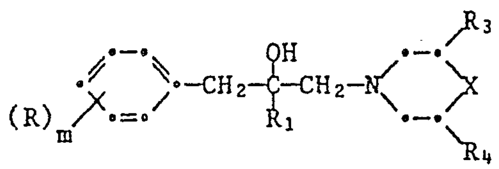

(II),

worin m, R, $R_1$, $R_3$, $R_4$ und X die für Formel I gegebene Bedeutung haben, an der Hydroxylgruppe verethern, acylieren bzw. carbamoylieren unter Einführung des Substituenten $R_2$ mit einem geeigneten Veretherungs-, Acylierungs- bzw. Carbamoylierungsmittel.

Verbindungen der Formel II können demgemäss mit einem Reaktanden der Formel III

$$R_2 - W \qquad (III)$$

umgesetzt werden, worin W eine übliche Abgangsgruppe, z.B. Halogen, Sulfonyloxy, oder Acyloxy darstellt, vorzugsweise in Gegenwart eines säurebindenden Mittels. Für Veretherungen und Carbamoylierungen kommen als Abgangsgruppe Halogen, bevorzugt Chlor oder Brom, in Frage. Für Acylierungen kommen als Abgangsgruppe Halogen, Sulfonyloxy oder Acyloxy (z.B. $-O-CO-R_5$) in Frage.

Es kann auch von Vorteil sein, die OH-Gruppe der Formel II in Form ihres Metallsalzes, besonders eines Alkali- oder Erdalkalimetallsalzes, einzusetzen.

Als säurebindende Mittel kommen (Erd)Alkalihydroxyde oder -carbonate in Frage, oder auch tert.Amine wie 4-Dimethylaminopyridin oder Trialkylamin.

Acylierungen zur Einführung eines Substituenten $-CO-R_5$ sind jedem Fachmann geläufig und werden mit entsprechenden Carbonsäurehalogeniden, besonders Bromiden und Chloriden, oder mit Carbonsäureanhydriden durchgeführt.

Als weitere Variante zur Herstellung von Verbindungen, worin $R_2$ einen Carbamoylrest $-CONHR_6$ bedeutet, lassen sich Verbindungen der Formel II mit Isocyanaten der Formel IV

$$O = C = N - R_6 \qquad (IV)$$

gegebenenfalls in Gegenwart eines Katalysators, wie z.B. 1,4-Diazabicyclo[2.2.2]octan zur Reaktion bringen.

Alkohole der Formel II lassen sich auch mit Alkoholen der Formel V

$$HO - R_2 \qquad (V)$$

in Gegenwart einer starken Säure (Schwefelsäure, Chlorwasserstoffsäure, u.a.) veräthern.

Bei dieser Kondensationsreaktion können die Reaktanden in einem geeigneten Lösungsmittel unter Rückfluss erhitzt werden, wobei gleichzeitig das entstehende Wasser azeotrop aus dem Reaktionsgemisch abdestilliert wird. Als Lösungsmittel kommen aromatische Kohlenwasserstoffe, wie Toluol oder der Alkohol V selbst in Frage. Bei dieser Reaktion arbeitet man zweckmässigerweise in Gegenwart einer starken Säure z.B. p-Toluolsulfonsäure.

Die vorstehenden Reaktionen lassen sich in einem weiten Temperaturbereich von -10°C bis +200°C (bzw. beim Siedepunkt des Lösungsmittels) durchführen.

Als Lösungsmittel dient vorzugsweise ein Ueberschuss des Reaktanden der Formel II oder V. Als reaktionsinerte Verdünnungsmittel können z.B. Chlorbenzol, Toluol, Nitrobenzol, Xylol, N,N-Dimethylformamid, N,N-Dimethylacetamid, Dimethylsulfoxid, Acetonitril oder Ether wie Tetrahydrofuran, Dioxan usw., oder aliphatische Kohlenwasserstoffe wie Cyclohexan, Hexan usw. anwesend sein.

B) Wenn $R_2$ nicht die Bedeutung $-COR_5$ oder $-CONHR_6$ hat, dann ist auch die Umsetzung eines Oxirans der Formel

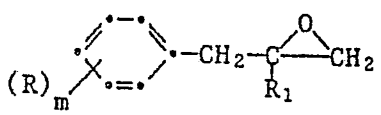

(VI),

mit einem Alkohol der Formel V

$$HO - R_2 \qquad (V)$$

bei einer Temperatur von -20° bis + 120°C in Gegenwart eines sauren Katalysators bzw. eines sauren Kondensationsmittel zu einem Glykolmonoether der Formel VII

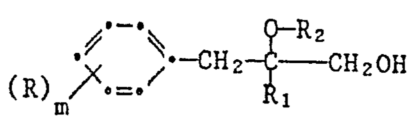

(VII),

möglich.

Anschliessend wird die Verbindung der Formel VII oder ein Ester davon in Gegenwart eines Säurebindemittels bzw. Kondensationsmittels bei einer Temperatur von 0° bis 150°C mit einer Verbindung der Formel VIII

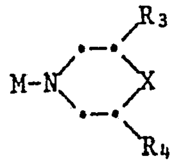

(VIII)

umgesetzt. M bedeutet in Formel VIII Wasserstoff oder bevorzugt ein Metallatom, insbesondere ein Alkalimetallatom, während die übrigen Substituenten die unter Formel I genannte Bedeutung haben.

Die Erfindung bezieht sich auch auf die bevorzugten neuen Oxiran-Derivate der Formel VI

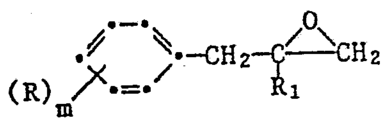

(VI),

worin

m = 1 oder 2,

R = Halogen; $C_1$-$C_6$-Alkyl; ein- oder mehrfach durch Halogen und/oder $C_1$-$C_3$-Alkoxy substituiertes $C_1$-$C_6$-Alkyl; $C_1$-$C_4$-Alkoxy; ein- oder mehrfach durch Halogen substituiertes $C_1$-$C_4$-Alkoxy; $C_3$-$C_6$-Cycloalkyl;

$R_1$ = $C_1$-$C_4$-Alkyl; ein- oder mehrfach durch Halogen substituiertes $C_1$-$C_4$-Alkyl,

mit der Masgabe, dass $R_1$ eine andere Bedeutung als Methyl hat, wenn m = 1 ist und R para-tert.Butyl bedeutet.

Es können Alkohole der Formel II durch direkte Reaktion eines Oxirans der Formel VI mit einem Amin der Formel VIII hergestellt werden.

Diese Reaktion wird vorteilhafterweise in Gegenwart eines Kondensationsmittels oder Säurebindemittels durchgeführt. Als solche kommen organische und anorganische Basen in Betracht z.B. tert.-

Amine; Pyridine; Alkali- und Erdalkalioxide, -hydride, -hydroxide, -carbonate und -hydrogencarbonate, Alkaliacetate sowie Alkalialkoholate.

Eine weitere Möglichkeit zur Synthese von Alkoholen der Formel II besteht darin, dass eine Grignard-Verbindung der Formel IX

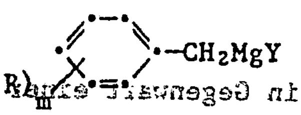
(IX),

mit einer Verbindung der Formel X

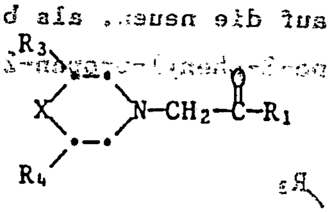
(X)

umgesetzt wird, oder dass

eine Grignard-Verbindung der Formel XI

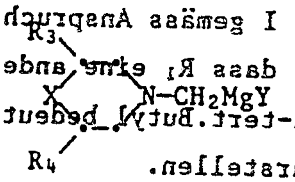
(XI)

mit einem Keton der Formel XII

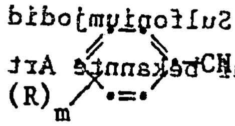
(XII)

zur Reaktion gebracht wird. Hierin haben die Substituenten R, X, $R_1$, $R_3$, $R_4$ und m die für Formel II gegebene Bedeutung, während Y Chlor oder Brom darstellt. Es reagiert also in beiden Fällen ein entsprechend strukturiertes Keton mit einem substituierten Methylmagnesiumhalogenid zu Verbindungen der Formel II.

BAD ORIGINAL

Eine weitere Möglichkeit zur Synthese von Verbindungen der Formel II ist die Anlagerung von Wasser an eine ungesättigte Verbindung der Formel XIII

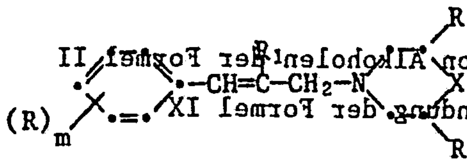

(XIII),

Die Reaktion wird bevorzugterweise in Gegenwart einer starken Säure als Katalysator durchgeführt.

Die Erfindung bezieht sich auch auf die neuen, als bevorzugte Zwischenprodukte dienenden 1-Amino-3-phenyl-propan-2-ol-Derivate der Formel II

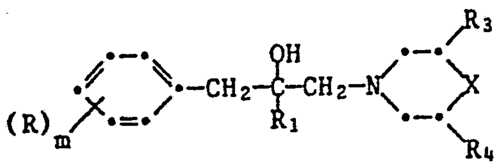

(II),

worin m = 1 oder 2 ist, und worin R, X, $R_1$, $R_3$ und $R_4$ die für Formel I gemäss Anspruch 1 gegebene Bedeutung haben, mit der Massgabe, dass $R_1$ eine andere Bedeutung als Methyl hat, wenn m = 1 ist, R para-tert.Butyl bedeutet, X für $CH_2$ steht und $R_3$ = $R_4$ = Wasserstoff darstellen.

Die Oxirane der Formel VI lassen sich aus den Ketonen der Formel XII nach der Corey-Synthese z.B. mit einem Sulfoniumjodid oder einem Sulfoxoniumjodid in Dimethylsulfoxid auf bekannte Art und Weise erhalten.

(J. Amer. Chem. Soc. 1965, 87, 1353 und
J. Amer. Chem. Soc. 1962, 84, 3782).

Die Ketone der Formel XII sind bekannt oder lassen sich nach bekannten Methoden herstellen.

BAD ORIGINAL

Die Propan-Derivate der Formel I besitzen in der 2-Stellung stets ein asymmetrisches *C-Atom und können daher in zwei enantiomeren Formen vorliegen. Im allgemeinen entsteht bei der Herstellung dieser Substanzen ein Gemisch beider Enantiomeren. Dieses lässt sich auf übliche Weise, z.B. durch fraktionierte Kristallisation von Salzen mit optisch aktiven, starken Säuren, in die reinen optischen Antipoden aufspalten. Beide Enantiomeren können unterschiedliche biologische Aktivitäten aufweisen.

Die vorliegende Erfindung betrifft alle reinen Enantiomeren bzw. Diastereomeren und deren Gemische untereinander.

Die aus der Literatur bekanntgewordenen strukturnächsten 3-Phenyl-propan-2-ol-Derivate stellen äusserst schwach wirksame Fungizide dar oder sind als Fungizide ungeeignet.

So wird in "Research Disclosure", No. 256, S. 410 (1985) die Verbindung 1-[3-(4-tert.Butylphenyl)-2-hydroxi-2-methylpropyl]-piperidin als Pflanzenfungizid beschrieben.

In der Eur. J. Chem.-Chim. Ther., Vol. 14(2), S. 165-170 (1979) werden 3-Phenyl-2-propanolamine als Pharmaceutica beschrieben, darunter das 1-(4H-Morpholino)-3-phenyl-2-ethyl-propan-2-ol.

Eine mikrobizide oder pflanzenfungizide Wirkung wird nicht angegeben.

Es wurde überraschend festgestellt, dass Verbindungen der Formel I, die entsprechende Ether der vorgenannten Verbindungen darstellen, im Gegensatz zu diesen ein für praktische Bedürfnisse sehr günstiges Mikrobizid-Spektrum gegen Pilze und Bakterien aufweisen. Sie besitzen sehr vorteilhafte kurative, präventive und systemische Eigenschaften und lassen sich zum Schutz von Kulturpflanzen einsetzen. Mit den Wirkstoffen der Formel I können Pflanzen oder an Pflanzenteilen (Früchte, Blüten, Laubwerk, Stengel, Knollen, Wurzeln) von unterschiedlichen Nutzkulturen die auftretenden

Mikroorganismen eingedämmt oder vernichtet werden, wobei auch später zuwachsende Pflanzenteile von derartigen Mikroorganismen verschont bleiben.

Die Wirkstoffe sind gegen die den folgenden Klassen angehörenden phytopathogenen Pilze wirksam: Ascomyceten (z.B. Venturia, Podosphaera, Erysiphe, Monilinia, Uncinula); Basidiomyceten (z.B. die Gattungen Hemileia, Rhizoctonia, Puccinia); Fungi imperfecti (z.B. Botrytis, Helmintosporium, Fusarium, Septoria, Cercospora und Alternaria). Ueberdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemässen Wirkstoffe zeichnen sich durch besonders gute Pflanzenverträglichkeit aus.

Die Erfindung betrifft somit auch mikrobizide Mittel sowie die Verwendung der Verbindungen der Formel I zur Bekämpfung pathogener Mikroorganismen, insbesondere pflanzenschädigender Pilze bzw. die präventive Verhütung eines Befalls an Pflanzen.

Als Zielkulturen für die hierin offenbarten Indikationsgebiete gelten im Rahmen dieser Erfindung z.B. folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben,

Hopfen, Bananen- und Naturkautschukgewächse sowie Zierpflanzen (Blumen, Sträucher, Laubbäume und Nadelbäume wie Koniferen). Diese Aufzählung stellt keine Limitierung dar.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Die Verbindungen der Formel I werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren werden gleich wie die Art der Mittel den angestrebten Zielen und den gegebenen Verhältnissen entsprechend gewählt. Im Agrarsektor liegen günstige Aufwandmengen im allgemeinen bei 50 g bis 5 kg Aktivsubstanz (AS) je ha; bevorzugt 100 g bis 2 kg AS/ha, insbesondere bei 200 g bis 1500 g AS/ha.

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Besonders vorteilhafte, applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, wie z.B. Phosphatidylethanolamin, Phosphatidylserin, Phosphatidylglycerin oder Lysolecithin.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Die in der Formulierungstechnik gebräuchlichen Tenside sind u.a. in folgenden Publikationen beschrieben:

"Mc Cutcheon's Detergents and Emulsifiers Annual"
BC Publishing Corp., Ringwood New Jersey, 1981;
Helmut Stache "Tensid-Taschenbuch" Carl Hanser-Verlag
München/Wien 1981.

M. and J. Ash. "Encyclopedia of Surfactants", Vol. I-III, Chemical Publishing Co., New York, 1980-1981.

Die agrochemischen Zubereitungen enthalten in der Regel 0,1 bis 99 %, insbesondere 0,1 bis 95 % Wirkstoff der Formel I, 99,9 bis 1 %, insbesondere 99,8 bis 5 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht.

Herstellungsbeispiele

Beispiel H1: Herstellung von

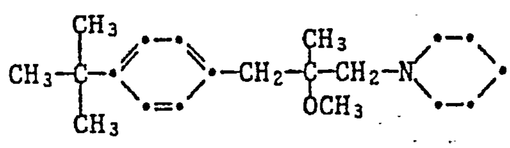

1-[3-(p-tert.Butylphenyl)-2-methyl-2-methoxypropyl]-piperidin.

Zu einer Suspension von 0,8 g 80%iger Natriumhydrid-Dispersion in 20 ml Dimethylformamid werden 5,8 g 1-[3-(p-tert.Butylphenyl)-2-methyl-2-hydroxypropyl]-piperidin, gelöst in 30 ml Dimethylformamid, innert 10 Min. zulaufen gelassen. Dann wird auf 40°C erwärmt und 5 Std. bei dieser Temperatur gerührt. Anschliessend wird zur roten Reaktionslösung bei 35°C innert 30 Min. 4,2 g Methyljodid zugetropft und 20 Std. bei 40°C gerührt. Nach dem Abkühlen auf Raumtemperatur wird das Reaktionsgemisch auf ~ 300 ml Wasser gegossen und 3x mit je 100 ml Ethylacetat extrahiert. Die organischen Phasen werden vereinigt, 3x mit je 150 ml Wasser gewaschen, über Natriumsulfat getrocknet, filtriert und das Lösungsmittel unter Vakuum verdampft.

Der flüssige Rückstand wird zur Reinigung mittels Ethylacetat/Hexan/-Methanol (4:3:1) über eine Kieselgelsäule chromatographiert. Die Titelverbindung wird als hellgelbes Oel erhalten, $n_D^{23}$: 1,5100.

Synthese der Zwischenprodukte

a) Herstellung von

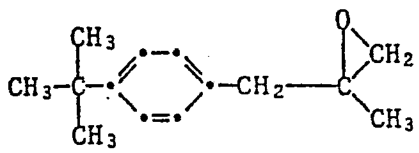

3-(p-tert.Butylphenyl)-2-methyl-1,2-epoxypropan.

4,0 g 80%ige Natriumhydrid-Dispersion werden in 90 ml Dimethylsulfoxid vorgelegt. Dann werden innert 2 Std. 31,9 g Trimethylsulfoxoniumjodid portionenweise zugegeben und 1 Std. bei Raumtemperatur nachgerührt. Anschliessend werden bei 20-25°C 23,5 g p-tert.Butylphenylaceton, gelöst in 100 ml Tetrahydrofuran, innert 3/4 Std. zugetropft. Das Reaktionsgemisch wird auf 55 bis 60°C erwärmt und 20 Std. bei dieser Temperatur gerührt, dann auf Raumtemperatur abgekühlt, auf ca. 500 ml 10 % Solelösung gegossen und 4x mit je 150 ml Ethylacetat extrahiert. Die organischen Phasen werden vereinigt, 3x mit je 100 ml Wasser gewaschen, über Natriumsulfat getrocknet, filtriert, und das Lösungsmittel wird unter Vakuum verdampft.

Die Verbindung wird als gelbes Oel erhalten.

b) Synthese von

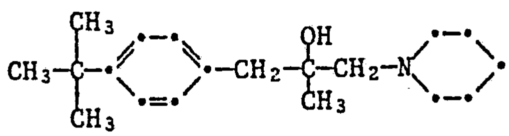

1-[3-(p-tert.Butylphenyl)-2-hydroxy-2-methylpropyl]-piperidin.

13,6 g 3-(p-tert.Butylphenyl)-2-methyl-1,2-epoxypropan, 12,0 g Piperidin und 1,6 g Kalium-tert.butylat werden in 150 ml Dimethylformamid auf 100°C erwärmt und 18 Std. bei dieser Temperatur gerührt. Anschliessend wird das Reaktionsgemisch auf Raumtemperatur abgekühlt, auf 300 ml 5%ige Solelösung gegossen und 4x mit je 100 ml Ethylacetat extrahiert. Die vereinigten organischen Phasen werden 3x mit je 200 ml Wasser gewaschen, über Natriumsulfat getrocknet, filtriert und das Lösungsmittel am Vakuum abgedampft. Der rote, flüssige Rückstand wird in 200 ml Diethylether gelöst und mit 3,65 ml 65%iger Salpetersäure versetzt. Der entstehende weisse Niederschlag wird abgenutscht, 2x mit je 100 ml Diethylether gewaschen, in 200 ml Wasser angeschlemmt, mit 15%iger Natronlauge neutralisiert und 3x mit je 100 ml Diethylether extrahiert. Die organischen Phasen werden vereinigt, 4x mit je 150 ml Wasser gewaschen, über Natriumsulfat getrocknet, filtriert und das Lösungsmittel verdampft.
Die Titelverbindung wird als Oel erhalten $n_D^{23}$: 1,5123.

Auf diese Art oder nach einer der weiter oben beschriebenen Methoden lassen sich folgende Zwischenprodukte herstellen:

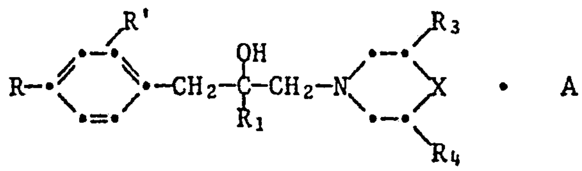

| R | R' | $R_1$ | X | $R_3$ | $R_4$ | A | Phys.Konstante |
|---|---|---|---|---|---|---|---|
| H | H | $-CH_3$ | $-CH_2-$ | H | H | --- | $n_D^{21}$: 1,5200 |
| Cl | Cl | $-CH_3$ | O | $-CH_3$ | $-CH_3$ | --- | $n_D^{22,5}$: 1,5342 |
| $t-C_4H_9$ | H | $-CH_3$ | $-CH_2-$ | H | H | $HNO_3$ | Smp. 135-139° |
| H | H | $-CF_3$ | $-CH_2-$ | H | H | --- | $n_D^{23}$ : 1,4788 |
| Cl | Cl | $-CH_3$ | $-CH_2-$ | H | H | --- | Smp. 61-63° |
| H | H | $-CF_3$ | O | $-CH_3$ | $-CH_3$ | $HNO_3$ | Smp. 118-119° |
| $t-C_4H_9$ | H | $-CH_3$ | $-CH_2-$ | H | H | --- | $n_D^{23}$ : 1,5123 |
| H | H | $-CF_3$ | $-CH_2-$ | H | H | $HNO_3$ | Smp. 108-125° |
| $t-C_4H_9$ | H | $-CH_3$ | O | $-CH_3$ | $-CH_3$ | --- | $n_D^{24,5}$: 1,5078 |
| $t-C_4H_9$ | H | $-CH_3$ | O | H | H | --- | $n_D^{27}$ : 1,5173 |
| $t-C_4H_9$ | H | $-CH_3$ | $-CH_2-$ | $-CH_3$ | $-CH_3$ | --- | $n_D^{26}$ : 1,5014 |
| $t-C_4H_9$ | H | $-CF_3$ | O | $-CH_3$ | $-CH_3$ | $HNO_3$ | Smp. 120-121° |
| $i-C_3H_7$ | H | $-CH_3$ | O | $-CH_3$ | $-CH_3$ | --- | $n_D^{26}$ : 1,5118 |
| $t-C_4H_9$ | H | $-CH_3$ | $-CH_2-$ | $-CH_3$ | H | --- | |
| $t-C_4H_9$ | H | $-CH_3$ | O | $-CH_3$ cis | $-CH_3$ cis | --- | $n_D^{25,5}$: 1,5028 |
| $t-C_4H_9$ | H | $-CH_3$ | O | $-CH_3$ trans | $-CH_3$ trans | --- | $n_D^{25,5}$: 1,5053 |
| $t-C_4H_9$ | H | $-CH_3$ | O | $-CH_3$ | $-CH_3$ | $HNO_3$ | Smp. 125-132° |
| $t-C_4H_9$ | H | $-CH_3$ | O | $-CH_3$ | $-CH_3$ | $HCl_3$ | Smp. 196-198° |
| $t-C_4H_9$ | H | $-C_2H_5$ | O | $-CH_3$ | $-CH_3$ | - | $n_D^{25,5}$ : 1,5047 |
| $t-C_4H_9$ | H | $-C_2H_5$ | $-CH_2-$ | H | H | - | $n_D^{25,5}$ : 1,5118 |
| R | R' | $R_1$ | X | $R_3$ | $R_4$ | A | Phys.Konstante |
| $t-C_4H_9$ | H | $-CH_3$ | $-CH_2-$ | $-CH_3$ | $-CH_3$ | $HNO_3$ | Smp. 100-105° |
| $t-C_4H_9$ | H | $-CH_3$ | $-CH_2-$ | $-CH_3$ | H | $HNO_3$ | Smp. 106-109° |

Es lassen sich folgende Verbindungen der Formel I herstellen:

Tabelle 1

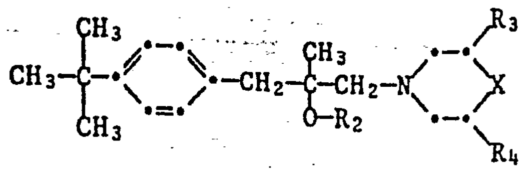

| Verb. Nr. | $R_2$ | $R_3$ | $R_4$ | X | Physikal. Konstante |
|---|---|---|---|---|---|
| 1.1 | $-CH_3$ | H | H | $CH_2$ | $n_D^{23}$: 1,5100 |
| 1.2 | $-CH_2C{\equiv}CH$ | $CH_3$ | $CH_3$ | O | $n_D^{22}$: 1,5143 |
| 1.3 | $-COCH_3$ | H | H | O | |
| 1.4 | $-CONHCH_3$ | $CH_3$ | $CH_3$ | O | |
| 1.5 | $-COCH_2OCH_3$ | H | H | $CH_2$ | |
| 1.6 | $-CH_3$ | $CH_3$ | $CH_3$ | O | $n_D^{23}$: 1,5018 |
| 1.7 | $COCH_2Cl$ | H | H | $CH_2$ | |
| 1.8 | -CONH- 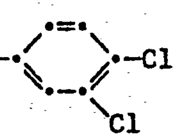 | H | H | $CH_2$ | |
| 1.9 | $-COCHCl_2$ | $CH_3$ | $CH_3$ | O | |
| 1.10 | $-C_4H_9-n$ | H | H | O | |
| 1.11 | $-C_3H_7-i$ | H | H | $CH_2$ | |
| 1.12 | $-COC_6H_5$ | H | H | $CH_2$ | |
| 1.13 | $-COCH{=}CH_2$ | $CH_3$ | $CH_3$ | O | |
| 1.14 | $-CH_2CH{=}CH_2$ | H | H | $CH_2$ | $n_D^{22}$: 1,5104 |
| 1.15 | $C_6H_{11}-n$ | $CH_3$ | $CH_3$ | O | |
| 1.16 | $-CH_2C_6H_5$ | $CH_3$ | $CH_3$ | $CH_2$ | |
| 1.17 | $-CONH-C_3H_7-i$ | H | H | $CH_2$ | |
| 1.18 | $-COCH_2Br$ | $CH_3$ | $CH_3$ | O | |
| 1.19 | $-CH_2-$ 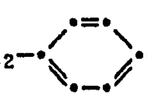 $-C_4H_9-t$ | H | H | $CH_2$ | |

Tabelle 1 (Fortsetzung):

| Verb. Nr. | $R_2$ | $R_3$ | $R_4$ | X | Physikal. Konstante |
|---|---|---|---|---|---|
| 1.20 | $C_2H_5$ | $CH_3$ | $CH_3$ | O | $n_D^{22,5}$: 1,4980 |
| 1.21 | $-CH_2C{\equiv}CH$ | H | H | $CH_2$ | |
| 1.22 | $-C_2H_5$ | H | H | $CH_2$ | $n_D^{25}$ : 1,5040 |
| 1.23 | $-CH_2CH_2Cl$ | H | H | O | |
| 1.24 | $-CH_3$ | $CH_3$ | $CH_3$ | $CH_2$ | $n_D^{26}$ : 1,4972 |
| 1.25 | 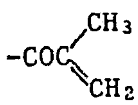 | H | H | $CH_2$ | |
| 1.26 | 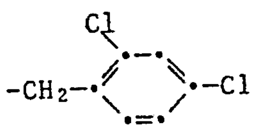 | H | H | $CH_2$ | |
| 1.27 | $-CH_3$ | $CH_3$ | H | $CH_2$ | $n_D^{26}$ : 1,5034 |
| 1.28 | $-CH_2CH_2OCH_3$ | H | H | $CH_2$ | |
| 1.29 | $-CONHCH_3$ | H | H | $CH_2$ | $n_D^{25}$ : 1,5131 |
| 1.30 | $-COCH_3$ | $CH_3$ | H | $CH_2$ | |
| 1.31 | $-CONHC_6H_5$ | H | H | $CH_2$ | |
| 1.32 | 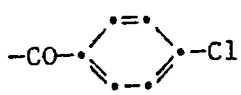 | $CH_3$ | $CH_3$ | O | |
| 1.33 | $-COCH_3$ | H | H | $CH_2$ | $n_D^{24}$: 1,5049 |
| 1.34 | $-CONH-C_2H_5$ | $CH_3$ | $CH_3$ | O | |
| 1.35 | $-COC_5H_{11}-n$ | H | H | $CH_2$ | |
| 1.36 | 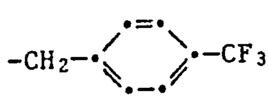 | $CH_3$ | $CH_3$ | O | |
| 1.37 | $-CONH-CH_3$ | $CH_3$ | $CH_3$ | $CH_2$ | |
| 1.38 | $-CH_2CH{=}CH_2$ | $CH_3$ | H | $CH_2$ | |
| 1.39 | $-CH_2C_6H_5$ | H | H | $CH_2$ | $n_D^{27}$: 1,5360 |
| 1.40 | $-COCH_3$ | $CH_3$ | $CH_3$ | O | $n_D^{23}$: 1,4978 |

Tabelle 1 (Fortsetzung):

| Verb. Nr. | $R_2$ | $R_3$ | $R_4$ | X | Physikal. Konstante |
|---|---|---|---|---|---|
| 1.41 | $-CH_2CH=CH_2$ | $CH_3$ | $CH_3$ | O | $n_D^{22,5}$: 1,5053 |
| 1.42 | $-CH_2-C_6H_5$ | $CH_3$ | $CH_3$ | O | $n_D^{20,5}$: 1,5263 |
| 1.43 | $-CH_3$ | $-CH_3$ | $-CH_3$ | O | $n_D^{24}$: 1,5013 |
| | | cis | | | |
| 1.44 | $-C_2H_5$ | $-CH_3$ | $-CH_3$ | O | $n_D^{24}$: 1,5070 |
| | | cis | | | |

| Verb. Nr. | $R_2$ | $R_3$ | $R_4$ | X | Salz | Physikal. Konstante |
|---|---|---|---|---|---|---|
| 1.45 | $-CH_2CH=CH_2$ | H | H | $CH_2$ | $HNO_3$ | Smp. 136-138°C |
| 1.46 | $-CH_2CH_3$ | $CH_3$ | $CH_3$ | $CH_2$ | - | |
| 1.47 | $-CH_3$ | $CH_3$ | $CH_3$ | O | $HNO_3$ | Smp. 115-117°C |
| 1.48 | $-CH_2C{\equiv}CH$ | $CH_3$ | H | $CH_2$ | - | |
| 1.49 | $-CH_2CH_3$ | H | H | $CH_2$ | $HNO_3$ | Smp. 134-135°C |
| 1.50 | $-CH_2CH=CH_2$ | $CH_3$ | $CH_3$ | $CH_2$ | - | |
| 1.51 | $-CH_2C_6H_5$ | H | H | $CH_2$ | $HNO_3$ | Smp. 147-149°C |
| 1.52 | $-CONHCH_3$ | $CH_3$ | H | $CH_2$ | - | |
| 1.53 | $-C_2H_5$ | $CH_3$ | H | $CH_2$ | - | |
| 1.54 | $-CH_2C{\equiv}CH$ | $CH_3$ | $CH_3$ | $CH_2$ | - | |
| 1.55 | $-CH_3$ | H | H | O | - | |
| 1.56 | $-CH_3$ | $CH_3$ | H | $CH_2$ | $HNO_3$ | Smp. 132-134°C |

0222694

sowie folgende Verbindungen der Formel I:

Tabelle 2:

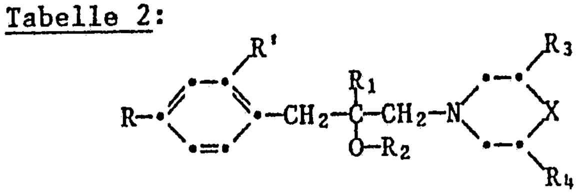

| Verb. Nr. | R | R' | $R_1$ | $R_2$ | $R_3$ | $R_4$ | X | Physikal. Konstante |
|---|---|---|---|---|---|---|---|---|
| 2.1 | $C_3H_7$-i | H | $CH_3$ | $CH_3$ | H | H | $CH_2$ | |
| 2.2 | Cl | Cl | $CH_3$ | $CH_3$ | H | H | $CH_2$ | |
| 2.3 | $C_3H_7$-i | H | $CF_3$ | $CH_3$ | H | H | $CH_2$ | |
| 2.4 | $OCHF_2$ | H | $CH_3$ | $CONHCH_3$ | H | H | $CH_2$ | |
| 2.5 | $C_4H_9$-t | H | $C_2H_5$ | $CH_3$ | $CH_3$ | $CH_3$ | O | Oel |
| 2.6 | $C_3H_7$-i | H | $CH_3$ | $CH_2C{\equiv}CH$ | H | H | $CH_2$ | |
| 2.7 | $C_4H_9$-t | H | $CF_3$ | $CH_2C_6H_5$ | H | H | $CH_2$ | |
| 2.8 | $C_3H_7$-i | H | $C_2H_5$ | $COCH_3$ | H | H | O | |
| 2.9 | $OC_4H_9$-t | H | $CH_3$ | $CH_3$ | $CH_3$ | $CH_3$ | O | |
| 2.10 | $C_4H_9$-t | H | $CF_3$ | CONH— 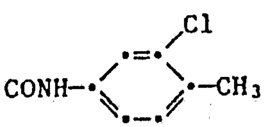 | H | H | $CH_2$ | |
| 2.11 | $C_3H_7$-i | H | $CH_3$ | $CH_3$ | $CH_3$ | $CH_3$ | O | |

Tabelle 2 (Fortsetzung):

| Verb. Nr. | R | R' | $R_1$ | $R_2$ | $R_3$ | $R_4$ | X | Physikal. Konstante |
|---|---|---|---|---|---|---|---|---|
| 2.12 | $C_4H_9$-t | H | $C_2H_5$ | $COCH_3$ | H | H | $CH_2$ | |
| 2.13 | $C_3H_7$-i | H | $CF_3$ | $CH_3$ | $CH_3$ | $CH_3$ | O | |
| 2.14 | Cyclohexyl | H | $CH_3$ | $CH_3$ | H | H | $CH_2$ | |
| 2.15 | $C_4H_9$-t | H | $CF_3$ | $CH_3$ | $CH_3$ | $CH_3$ | O | $n_D^{23}$: 1,4770 |
| 2.16 | $C_3H_7$-i | H | $CH_3$ | $CH_2C_6H_5$ | H | H | $CH_2$ | |
| 2.17 | $C_4H_9$-t | H | $C_2H_5$ | $CONHCH_3$ | H | H | $CH_2$ | |
| 2.18 | Cl | Cl | $CH_3$ | $CH_3$ | $CH_3$ | $CH_3$ | O | |
| 2.19 | $C_3H_7$-i | H | $CH_3$ | $CONHC_6H_5$ | H | H | $CH_2$ | |
| 2.20 | $C_4H_9$-t | H | $CF_3$ | $CH_3$ | H | H | $CH_2$ | |
| 2.21 | $C_3H_7$-i | H | $CH_3$ | $CONHCH_3$ | $CH_3$ | $CH_3$ | $CH_2$ | |
| 2.22 | $OC_4H_9$-t | H | $CH_3$ | $CH_3$ | H | H | $CH_2$ | |
| 2.23 | $C_4H_9$-t | H | $CF_3$ | $COCH_3$ | H | H | $CH_2$ | |
| 2.24 | $C_3H_7$-i | H | $CH_3$ | $CH_3$ | $CH_3$ | H | $CH_2$ | |
| 2.25 | Cl | Cl | $CH_3$ | $CONHC_3H_7$-i | H | H | O | |
| 2.26 | $C_4H_9$-t | H | $CF_3$ | $CH_2CH=CH_2$ | H | H | $CH_2$ | |
| 2.27 | $C_3H_7$-i | H | $CF_3$ | $COCH_3$ | $CH_3$ | $CH_3$ | O | |

Tabelle 2 (Fortsetzung):

| Verb. Nr. | R | R' | $R_1$ | $R_2$ | $R_3$ | $R_4$ | X | Physikal. Konstante |
|---|---|---|---|---|---|---|---|---|
| 2.28 | $OC_4H_9$-t | H | $CH_3$ | $C_2H_5$ | $CH_3$ | $CH_3$ | O | |
| 2.29 | $C_4H_9$-t | H | $CF_3$ | $CONHCH_3$ | H | H | $CH_2$ | |
| 2.30 | $C_3H_7$-i | H | $CF_3$ | $CH_2$— 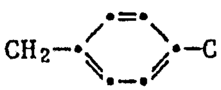 —$C_4H_9$-t | H | H | $CH_2$ | |
| 2.31 | $C_4H_9$-t | H | $CF_3$ | $CH_2$— 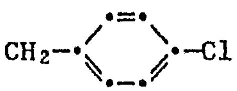 —Cl | H | H | $CH_2$ | |
| 2.32 | $OCHF_2$ | H | $CH_3$ | $CH_3$ | $CH_3$ | $CH_3$ | O | |
| 2.33 | Cyclohexyl | H | $CH_3$ | $COCH_3$ | $CH_3$ | $CH_3$ | O | |
| 2.34 | $C_4H_9$-t | H | $C_2H_5$ | $CH_3$ | H | H | $CH_2$ | |
| 2.35 | $C_3H_7$-i | H | $CH_3$ | $CH_3$ | $CH_3$ | $CH_3$ | $CH_2$ | |
| 2.36 | $C_4H_9$-t | H | $CF_3$ | CONH-$C_4H_9$-i | H | $CH_3$ | $CH_2$ | |
| 2.37 | $C_4H_9$-t | H | $CF_3$ | $COCH_2Cl$ | H | H | $CH_2$ | |
| 2.38 | $C_3H_7$-i | H | $CH_3$ | $COCH_2OCH_3$ | H | H | $CH_2$ | |
| 2.39 | $C_4H_9$-t | H | $CF_3$ | $COCH{=}CH_2$ | $CH_3$ | $CH_3$ | O | |
| 2.40 | $C_4H_9$-t | H | $CF_3$ | $CH_3$ | $CH_3$ | H | $CH_2$ | |
| 2.41 | $C_4H_9$-t | H | $CF_3$ | $CH_3$ | $CH_3$ | $CH_3$ | $CH_2$ | |
| 2.42 | Cyclohexyl | H | $CH_3$ | $CH_3$ | $CH_3$ | $CH_3$ | O | |

Das $HNO_3$-Salz der Verbindung Nr. 2.15 besitzt einen Schmelzpunkt von 108-110°C.

Das $HNO_3$-Salz der Verbindung Nr. 2.5 besitzt einen Schmelzpunkt von 115-116°C.

Formulierungsbeispiele für Wirkstoffe der Formel I
(% = Gewichtsprozent)

| F1. Lösungen | a) | b) | c) | d) |
|---|---|---|---|---|
| Wirkstoff aus den Tabellen | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

(MG = Molekulargewicht)

Die Lösungen sind zur Anwendung in Form kleinster Tropfen geeignet.

| F2. Granulate | a) | b) |
|---|---|---|
| Wirkstoff aus den Tabellen | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| F3. Stäubemittel | a) | b) |
|---|---|---|
| Wirkstoff aus den Tabellen | 2 % | 5 % |
| Hochdisperse Kieselsäure | 1 % | 5 % |
| Talkum | 97 % | - |
| Kaolin | - | 90 % |

Durch inniges Vermischen auf Trägerstoffe mit dem Wirkstoff erhält man gebrauchsfertige Stäubemittel.

| F4. Spritzpulver | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F5. Emulsions-Konzentrat | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| F6. Umhüllungs-Granulat | |
|---|---|
| Wirkstoff aus den Tabellen | 3 % |
| Polyethylenglykol (MG 200) | 3 % |
| Kaolin | 94 % |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

**Biologische Beispiele:**

Im folgenden werden die aus der Literatur bekannten strukturnächsten 1-Amino-3-phenyl-propan-2-ol Derivate zum Vergleich herangezogen. Dabei bedeuten:

Verbindung F = 1-[3-(4-tert.-Butylphenyl)-2-hydroxi-2-methyl-propyl]-piperidin,

Verbindung G = 1-(4H-Morpholino)-3-phenyl-2-ethylpropan-2-ol.

**Beispiel B1: Wirkung gegen Puccinia graminis auf Weizen**

a) Residual-protektive Wirkung

Weizenpflanzen wurden 6 Tage nach der Aussaat mit einer aus Spritzpulver des Wirkstoffes hergestellten Spritzbrühe (0,02 % Aktivsubstanz) besprüht. Nach 24 Stunden wurden die behandelten Pflanzen mit einer Uredosporensuspension des Pilzes infiziert. Nach einer Inkubation während 48 Stunden bei 95-100 % relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

b) Systemische Wirkung

Zu Weizenpflanzen wurde 5 Tage nach der Aussaat eine aus Spritzpulver des Wirkstoffes hergestellte Spritzbrühe gegossen (0,006 % Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95-100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

BAD ORIGINAL

Unbehandelte aber infizierte Kontrollpflanzen zeigten einen Puccinia-Befall von 100 %. Verbindungen aus den Tabellen 1 und 2 zeigten gegen Puccinia-Pilze eine gute Wirkung. Unter anderen hemmten die Verbindungen Nr. 1.1, 1.2, 1.6, 1.14, 1.20, 1.24, 1.27, 1.40, 1.43, 1.44, 2.5, 2.15, 2.20 den Puccinia-Befall auf 0 bis 5 %.

Verbindung F: 20-50 % Befall (leichte Phytotoxizität an der Pflanze),

Verbindung G: > 50 % Befall (= wirkungslos).

Beispiel B2: Wirkung gegen Cercospora arachidicola auf Erdnusspflanzen

a) Residual-protektive Wirkung

10-15 cm hohe Erdnusspflanzen wurden mit einer aus Spritzpulver der Wirksubstanz hergestellten Spritzbrühe (0,02 % Aktivsubstanz) besprüht und 48 Stunden später mit einer Konidiensuspension des Pilzes infiziert. Die infizierten Pflanzen wurden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgte 12 Tage nach der Infektion basierend auf Anzahl und Grösse der auftretenden Flecken.

b) Systemische Wirkung

Zu 10-15 cm hohen Erdnusspflanzen wurde eine aus Spritzpulver des Wirkstoffes hergestellte Spritzbrühe gegossen (0,06 % Aktivsubstanz bezogen auf das Erdvolumen). Nach 48 Stunden wurden die behandelten Pflanzen mit einer Konidiensuspension des Pilzes infiziert und 72 Stunden bei ca. 21°C und hoher Luftfeuchtigkeit inkubiert. Anschliessend wurden die Pflanzen im Gewächshaus aufgestellt und nach 11 Tagen der Pilzbefall beurteilt.

Im Vergleich zu unbehandelten aber infizierten Kontrollpflanzen (Anzahl und Grösse der Flecken = 100 %), zeigten Erdnusspflanzen, die mit Wirkstoffen aus den Tabellen 1 und 2 behandelt wurden, einen stark reduzierten Cercospora-Befall. So verhinderten die Verbindun-

gen Nr. 1.1, 1.6, 1.22, 1.24, 1.40, 1.43, 2.5, 2.15, 2.20, und 2.42 in obigen Versuchen das Auftreten von Flecken fast vollständig (0-10 %),

Verbindung F: > 50 % Befall;
Verbindung G: > 50 % Befall.

Beispiel B3: Wirkung gegen Erysiphae graminis auf Gerste

a) Residual-protektive Wirkung

Ca. 8 cm hohe Gerstenpflanzen wurden mit einer aus Spritzpulver des Wirkstoffes hergestellten Spritzbrühe (0,02 % Aktivsubstanz) besprüht. Nach 3-4 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,006 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

Verbindungen der Formel I zeigten eine gute Wirkung gegen Erysiphe-Pilze. Unbehandelte, aber infizierte Kontrollpflanzen zeigten einen Erysiphe-Befall von 100 %. Unter anderen Verbindungen aus den Tabellen 1 und 2 hemmten die Verbindungen Nr. 1.1, 1.6, 1.14, 1.20, 1.24, 1.27, 1.29, 1.40, 1.43, 1.44, 2.5, 2.13, 2.14, 2.15, 2.20, 2.24, 2.41 und 2.42 den Pilzbefall bei Gerste auf 0 bis 5 %;

Verbindung F: 20-50 % Befall;
Verbindung G: > 50 % Befall.

<u>Beispiel B4</u>: <u>Residual-protektive Wirkung gegen Venturia inaequalis auf Apfeltrieben</u>.

Apfelstecklinge mit 10-20 cm langen Frischtrieben wurden mit einer aus Spritzpulver des Wirkstoffes hergestellten Spritzbrühe (0,06 % Aktivsubstanz) besprüht. Nach 24 Stunden wurden die behandelten Pflanzen mit einer Konidiensuspension des Pilzes infiziert. Die Pflanzen wurden dann während 5 Tagen bei 90-100 % relativer Luftfeuchtigkeit inkubiert und während 10 weiteren Tagen in einem Gewächshaus bei 20-24°C aufgestellt. Der Schorfbefall wurde 15 Tage nach der Infektion beurteilt. Verbindungen aus den Tabellen 1 und 2 bewirkten eine deutliche Hemmung des Krankheitsbefalls. So hemmten die Verbindungen 1.1, 1.6, 1.20, 1.22, 1.24, 1.40, 1.43, 2.15, 2.20 und 2.24 den Pilzbefall fast vollständig (0-5 %). Unbehandelte aber infizierte Triebe zeigten einen 100 %igen Venturia-Befall, desgleichen Triebe, die mit Verbindung F oder G behandelt worden waren.

<u>Beispiel B5</u>: <u>Wirkung gegen Botrytis cinerea auf Bohnen</u>

<u>Residual protektive Wirkung</u>

Ca. 10 cm hohe Bohnen-Pflanzen wurden mit einer aus Spritzpulver des Wirkstoffes hergestellten Spritzbrühe (0,02 % Aktivsubstanz) besprüht. Nach 48 Stunden wurden die behandelten Pflanzen mit einer Konidiensuspension des Pilzes infiziert. Nach einer Inkubation der infizierten Pflanzen während 3 Tagen bei 95-100 % relativer Luftfeuchtigkeit und 21°C erfolgte die Beurteilung des Pilzbefalls. Der Botrytis-Befall unbehandelter aber infizierter Bohnenpflanzen betrug 100 %. Der Befall nach Behandlung mit einer der Verbindungen der Formel I betrug <20 %; bei Behandlung mit den Verbindungen Nr. 1.2, 1.6, 1.20, 1.43, 1.44 und anderen trat kein Befall auf (0-5 %);

<u>Verbindung F</u>: 10-20 % Befall;

<u>Verbindung G</u>: > 50 % Befall.

Patentansprüche:

1. Propan-2-ol-Derivate der Formel I,

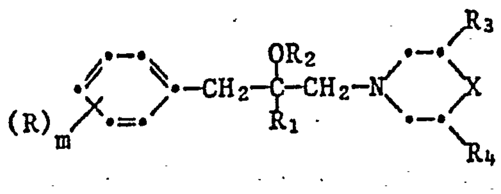

(I),

worin

m = 0 bis 3,

R = Halogen; $C_1$-$C_6$-Alkyl; ein- oder mehrfach durch Halogen und/oder $C_1$-$C_3$-Alkoxy substituiertes $C_1$-$C_6$-Alkyl; $C_1$-$C_4$-Alkoxy; ein- oder mehrfach durch Halogen substituiertes $C_1$-$C_4$-Alkoxy; $C_3$-$C_6$-Cycloalkyl;

$R_1$ = $C_1$-$C_4$-Alkyl; ein- oder mehrfach durch Halogen substituiertes $C_1$-$C_4$-Alkyl,

$R_2$ = $C_1$-$C_6$-Alkyl; ein- oder mehrfach durch Halogen und/oder $C_1$-$C_3$-Alkoxy substituiertes $C_1$-$C_6$-Alkyl; $C_2$-$C_4$-Alkenyl; 2-Propinyl; 3-Halogen-2-propinyl; Benzyl; ein- oder mehrfach durch Halogen, $C_1$-$C_4$-Alkoxy, $C_1$-$C_4$-Alkyl, $C_1$-$C_4$-Haloalkyl oder $C_1$-$C_4$-Haloalkoxy substituiertes Benzyl,

oder $-\overset{O}{\overset{\|}{C}}-R_5$ oder $-\overset{O}{\overset{\|}{C}}NH-R_6$ bedeuten, wobei

$R_5$ = $C_1$-$C_6$-Alkyl; ein- oder mehrfach durch Halogen und/oder $C_1$-$C_3$-Alkoxy substituiertes $C_1$-$C_6$-Alkyl; $C_2$-$C_4$-Alkenyl; Phenyl; Benzyl; ein- oder mehrfach durch Halogen, $C_1$-$C_4$-Alkyl, $C_1$-$C_4$-Alkoxy, $C_1$-$C_4$-Haloalkyl, $C_1$-$C_4$-Haloalkoxy substituiertes Phenyl oder Benzyl und

$R_6$ = $C_1$-$C_6$-Alkyl; $C_2$-$C_4$-Alkenyl; Phenyl; ein- oder mehrfach durch Halogen, $C_1$-$C_4$-Alkyl, $C_1$-$C_4$-Alkoxy, $C_1$-$C_4$-Haloalkoxy, $C_1$-$C_4$-Haloalkyl substituiertes Phenyl sind, und

$R_3$ und $R_4$ unabhängig H oder $C_1$-$C_4$-Alkyl darstellen, während

X = Sauerstoff oder $CH_2$ bedeutet,

unter Einschluss ihrer Säureadditionssalze.

2. Verbindungen der Formel I gemäss Anspruch 1, worin X = O und m = 1 oder 2 sind,

R = Halogen, $C_1$-$C_4$-Alkyl, $C_1$-$C_2$-Alkoxy, ein- bis dreifach durch Halogen oder $C_1$-$C_3$-Alkoxy substituiertes Methyl; ein- bis dreifach durch Halogen substituiertes $C_1$-$C_2$-Alkoxy; $C_3$-$C_6$-Cycloalkyl ist;

$R_1$ = Methyl, Ethyl oder Isopropyl bedeutet, die unsubstituiert oder ein- oder mehrfach durch Fluor oder Chlor substituiert sind; während

$R_2$ die für Formel I gegebene Bedeutung hat, und

$R_3$ und $R_4$ Wasserstoff, Methyl oder Ethyl darstellen.

3. Verbindungen der Formel I gemäss Anspruch 2, worin

$R_2$ = $C_1$-$C_4$-Alkyl, ein- oder mehrfach durch Halogen substituiertes $C_1$-$C_4$-Alkyl, durch eine $C_1$-$C_3$-Alkoxy-Gruppe substituiertes $C_1$-$C_2$-Alkyl; $C_2$-$C_4$-Alkenyl; 2-Propinyl; Benzyl oder bis zu dreifach durch Chlor und/oder Methyl substituiertes Benzyl, oder -CO-$R_5$ bedeutet, wobei

$R_5$ = $C_1$-$C_4$-Alkyl, ein- oder mehrfach durch F, Cl oder Br substituiertes $C_1$-$C_3$-Alkyl, durch eine $C_1$-$C_3$-Alkoxy-Gruppe substituiertes $C_1$-$C_3$-Alkyl; Allyl oder Methylallyl; oder Phenyl darstellt, das bis zu dreifach durch Halogen, Methyl, Methoxy oder $CF_3$ substituiert sein kann.

4. Verbindungen der Formel I gemäss Anspruch 2, worin m = 1 oder 2 ist,

R = Fluor, Chlor oder Brom, Methyl, Ethyl, Isopropyl oder tert.-Butyl, $C_1$-$C_2$-Alkoxy, ein- bis dreifach durch Fluor und/oder Chlor substituiertes Methyl, durch $C_1$-$C_3$-Alkoxy substituiertes Methyl oder ein- bis dreifach durch Fluor oder Chlor substituiertes $C_1$-$C_2$-Alkoxy oder $C_3$-$C_6$ Cycloalkyl bedeutet;

$R_1$ = Methyl, Ethyl oder $CF_3$ ist,

$R_2$ = $C_1$-$C_4$-Alkyl, ein- bis dreifach durch Fluor oder Chlor substituiertes $C_1$-$C_4$-Alkyl, durch $C_1$-$C_3$-Alkoxy substituiertes $C_1$-$C_4$-Alkyl; $C_2$-$C_4$-Alkenyl; 2-Propinyl; Benzyl; ein- bis dreifach durch Chlor und/oder Methyl substituiertes Benzyl, oder -CO-$R_5$ oder -CONH$R_6$ ist, wobei

$R_5$ = Methyl, Ethyl, Isopropyl, ein- bis dreifach durch F oder Cl substituiertes $C_1$-$C_3$-Alkyl, durch Methoxy, Ethoxy oder Isopropoxy substituiertes Methyl oder Ethyl; Allyl oder Methylallyl; oder Phenyl darstellt, das bis zu dreifach durch Halogen, Methyl, Methoxy oder $CF_3$ substituiert sein kann; und

$R_6$ = $C_1$-$C_6$-Alkyl oder unsubstituiertes oder bis zu dreifach durch Halogen, Methyl, Methoxy oder $CF_3$ substituiertes Phenyl bedeutet; und

$R_3$ und $R_4$ Wasserstoff oder Methyl darstellen.

5. Verbindungen der Formel I gemäss Anspruch 1, worin X = O ist, $R_3$ und $R_4$ cis-ständig zueinander sind und je $C_1$-$C_4$-Alkyl bedeuten.

6. Verbindungen der Formel I gemäss Anspruch 1, worin X = $CH_2$ und m = 1 oder 2 sind;

R = Halogen, $C_1$-$C_4$-Alkyl, $C_1$-$C_2$-Alkoxy, ein- bis dreifach durch Halogen oder $C_1$-$C_3$-Alkoxy substituiertes Methyl; ein- bis dreifach durch Halogen substituiertes $C_1$-$C_2$-Alkoxy; oder $C_3$-$C_6$-Cycloalkyl bedeutet;

$R_1$ = Methyl, Ethyl oder Isopropyl, die unsubstituiert oder ein- oder mehrfach durch Fluor oder Chlor substituiert sind; während

$R_2$ die für Formel I gegebene Bedeutung hat, und

$R_3$ und $R_4$ Wasserstoff, Methyl oder Ethyl darstellen

7. Verbindungen der Formel I gemäss Anspruch 6, worin

$R_2$ = $C_1$-$C_4$-Alkyl, ein- oder mehrfach durch Halogen substituiertes $C_1$-$C_4$-Alkyl, durch eine $C_1$-$C_3$-Alkoxy-Gruppe substituiertes $C_1$-$C_2$-Alkyl; $C_2$-$C_4$-Alkenyl; 2-Propinyl; Benzyl oder bis zu dreifach durch Chlor und/oder Methyl substituiertes Benzyl, oder -CO-$R_5$ bedeutet, wobei

$R_5$ = $C_1$-$C_4$-Alkyl, ein- oder mehrfach durch F, Cl oder Br substituiertes $C_1$-$C_3$-Alkyl, durch eine $C_1$-$C_3$-Alkoxy-Gruppe substituiertes $C_1$-$C_3$-Alkyl; Allyl oder Methylallyl; oder Phenyl darstellt, das bis zu dreifach durch Halogen, Methyl, Methoxy oder $CF_3$ substituiert sein kann.

8. Verbindungen der Formel I gemäss Anspruch 6, worin m = 1 oder 2,

R = Fluor, Chlor oder Brom, Methyl, Ethyl, Isopropyl oder tert.-Butyl, $C_1$-$C_2$-Alkoxy, ein- bis dreifach durch Fluor und/oder Chlor substituiertes Methyl, durch $C_1$-$C_3$-Alkoxy substituiertes Methyl oder ein- bis dreifach durch Fluor oder Chlor substituiertes $C_1$-$C_2$-Alkoxy; oder $C_3$-$C_6$-Cycloalkyl bedeutet;

$R_1$ = Methyl, Ethyl oder $CF_3$ ist;

$R_2$ = $C_1$-$C_4$-Alkyl, ein- bis dreifach durch Fluor oder Chlor substituiertes $C_1$-$C_4$-Alkyl, durch $C_1$-$C_3$-Alkoxy substituiertes $C_1$-$C_4$-Alkyl; $C_2$-$C_4$-Alkenyl; 2-Propinyl; Benzyl; ein- bis dreifach durch Chlor und/oder Methyl substituiertes Benzyl, oder -CO-$R_5$ oder -CONH$R_6$, wobei

$R_5$ = Methyl, Ethyl, Isopropyl, ein- bis dreifach durch F oder Cl substituiertes $C_1$-$C_3$-Alkyl, durch Methoxy, Ethoxy oder Isopropoxy substituiertes Methyl oder Ethyl; Allyl oder Methylallyl; oder Phenyl darstellt, das bis zu dreifach durch Halogen, Methyl, Methoxy oder $CF_3$ substituiert sein kann; und

$R_6$ = $C_1$-$C_6$-Alkyl oder unsubstituiertes oder bis zu dreifach durch Halogen, Methyl, Methoxy oder $CF_3$ substituiertes Phenyl bedeutet; und

$R_3$ und $R_4$ Wasserstoff oder Methyl darstellen.

9. 1-(4H-Morpholino)-3-(4-tert.butylphenyl)-2-methyl-2-methoxypropan;
1-(4H-2,6-Dimethylmorpholino)-3-(4-tert.butylphenyl)-2-methyl-2-methoxypropan;
1-(4H-2,6-Dimethylmorpholino)-3-(4-tert.-butylphenyl)-2-methyl-2-ethoxypropan;
1-(cis-4H-2,6-Dimethylmorpholino)-3-(4-tert.butylphenyl)-2-methyl-2-methoxypropan;

1-(cis-4H-2,6-Dimethylmorpholino)-3-(4-tert.butylphenyl)-2-methyl-2-ethoxypropan;
1-(4H-2,6-Dimethylmorpholino)-3-(4-tert.-butylphenyl)-2-methyl-2-propargyloxypropan;
1-(4H-2,6-Dimethylmorpholino)-3-(4-tert.-butylphenyl)-2-methyl-2-allyloxypropan.

10. 1-(1H-Piperidino)-3-(4-tert.butylphenyl)-2-methyl-2-methoxypropan;
1-(1H-Piperidino)-3-(4-tert.butylphenyl)-2-methyl-2-ethoxypropan;
1-(1H-Piperidino)-3-(4-tert.butylphenyl)-2-methyl-2-allyloxypropan;
1-(1H-Piperidino)-3-(4-tert.butylphenyl)-2-ethyl-2-methoxypropan.

11. 1-(1H-3,5-Dimethylpiperidino)-3-(4-tert.-butylphenyl)-2-methyl-2-methoxypropan;
1-(1H-3-Methylpiperidino)-3-(4-tert.-butylphenyl)-2-methyl-2-methoxypropan.

12. Verfahren zur Herstellung von Verbindungen der Formel I gemäss Anspruch 1 durch Reaktion einer Verbindung der Formel II

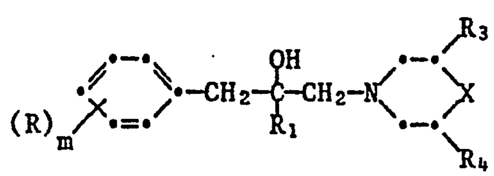

(II),

worin m, R, $R_1$, $R_3$, $R_4$ und X die für Formel I gegebene Bedeutung haben, an der Hydroxylgruppe mit einem zur Einführung des Substituenten $R_2$ geeigneten Veretherungs-, Acylierungs- oder Carbamoylierungsmittel, wobei $R_2$ die für Formel I gegebene Bedeutung hat.

13. Verfahren gemäss Anspruch 12, wobei zur Einführung des Substituenten $R_2$ ein Reaktand der Formel III

$$R_2 - W \qquad (III)$$

verwendet wird, worin W eine der Abgangsgruppen Halogen, Sulfonyloxy oder Acyloxy bedeutet.

14. Mikrobizides Mittel zur Bekämpfung oder Verhütung eines Befalls durch Mikroorganismen, dadurch gekennzeichnet, dass es als mindestens eine aktive Komponente eine Verbindung der Formel I gemäss Anspruch 1, zusammen mit einem geeigneten Trägermaterial enthält.

15. Verwendung von Verbindungen der Formel I gemäss Anspruch 1 zur Bekämpfung und/oder zur präventiven Verhütung eines Befalls von Mikroorganismen.

16. Verfahren zur Bekämpfung oder Verhütung eines Befalls von Kulturpflanzen durch phytopathogene Mikroorganismen, dadurch gekennzeichnet, dass man eine Verbindung der Formel I gemäss Anspruch 1 auf die Pflanze, auf ihren Standort oder auf Teile der Pflanze appliziert.

17. Oxiran-Derivate der Formel VI

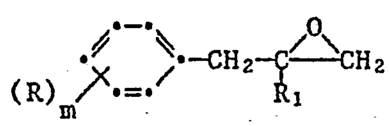

(VI),

worin

m = 1 oder 2,

R = Halogen; $C_1$-$C_6$-Alkyl; ein- oder mehrfach durch Halogen und/oder $C_1$-$C_3$-Alkoxy substituiertes $C_1$-$C_6$-Alkyl; $C_1$-$C_4$-Alkoxy; ein- oder mehrfach durch Halogen substituiertes $C_1$-$C_4$-Alkoxy; $C_3$-$C_6$-Cycloalkyl; und

$R_1$ = $C_1$-$C_4$-Alkyl oder ein- oder mehrfach durch Halogen substituiertes $C_1$-$C_4$-Alkyl darstellen,

mit der Massgabe, dass $R_1$ eine andere Bedeutung als Methyl hat, wenn m = 1 ist und R para-tert.Butyl bedeutet.

18. 1-Amino-3-phenyl-propan-2-ol-Derivate der Formel II

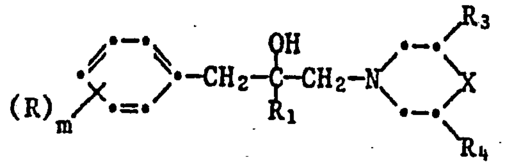

(II),

worin m = 1 oder 2 ist, und worin
R, $R_1$, $R_3$ und $R_4$ die für Formel I gemäss Anspruch 1 gegebene Bedeutung haben, mit der Massgabe, dass $R_1$ eine andere Bedeutung als Methyl hat, wenn m = 1 ist, R para-tert.Butyl bedeutet, X für $CH_2$ steht und $R_3$ = $R_4$ = Wasserstoff darstellen.

7.5/PK/eg*/sm*/we*